(19) **Europäisches Patentamt**
**European Patent Office**
**Office européen des brevets**

(11) **EP 2 579 037 B1**

(12) **EUROPEAN PATENT SPECIFICATION**

(45) Date of publication and mention
of the grant of the patent:
**12.08.2015 Bulletin 2015/33**

(51) Int Cl.:
***G01N 33/50*** (2006.01)

(21) Application number: **11789426.1**

(22) Date of filing: **27.05.2011**

(86) International application number:
**PCT/JP2011/002979**

(87) International publication number:
**WO 2011/152012 (08.12.2011 Gazette 2011/49)**

(54) **METHOD FOR TESTING THE SEVERITY OF AN ILLNESS**

VORRICHTUNG ZUM TESTEN DER SCHWERE EINER KRANKHEIT

PROCÉDÉ POUR ANALYSER LA GRAVITÉ D'UNE MALADIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2010 JP 2010125354**

(43) Date of publication of application:
**10.04.2013 Bulletin 2013/15**

(73) Proprietor: **The University of Tokushima Tokushima-shi,
Tokushima 770-8501 (JP)**

(72) Inventors:
• **KIDO, Hiroshi**
**Tokushima-shi, Tokushima 770-8503 (JP)**
• **NISHIMURA, Masaji**
**Tokushima-shi, Tokushima 770-8503 (JP)**
• **CHIDA, Junji**
**Tokushima-shi, Tokushima 770-8503 (JP)**

(74) Representative: **Nederlandsch Octrooibureau
P.O. Box 29720
2502 LS The Hague (NL)**

(56) References cited:
**EP-A1- 1 650 563        WO-A1-98/48044
WO-A1-2009/096429    WO-A1-2009/096429
JP-A- 2005 505 306    JP-A- 2009 519 467
JP-A- 2009 527 567**

• **JABS ET AL: "Plasma levels of high-energy compounds compared with severity of illness in critically ill patients in the intensive care unit", SURGERY, C.V. MOSBY CO., ST. LOUIS, US, vol. 124, no. 1, 1 July 1998 (1998-07-01), pages 65-72, XP022421569, ISSN: 0039-6060**
• **SCHUSTER ET AL: "Prognostic value of blood lactate in critically ill patients", RESUSCITATION, ELSEVIER, IE, vol. 11, no. 3-4, 1 March 1984 (1984-03-01), pages 141-146, XP026331888, ISSN: 0300-9572, DOI: 10.1016/0300-9572(84)90011-X [retrieved on 1984-03-01]**
• **MASAYUKI TAKAHASHI ET AL: "Arterial ketone body ratio as a prognostic indicator in acute heart failure", JOURNAL OF LABORATORY AND CLINICAL MEDICINE, vol. 129, no. 1, 1 January 1997 (1997-01-01), pages 72-80, XP055078919, ISSN: 0022-2143, DOI: 10.1016/S0022-2143(97)90163-3**
• **YAMAMOTO Y ET AL: "Prognostic implications of postoperative suppression of arterial ketone body ratio: time factor involved in the suppression of hepatic mitochondrial oxidation-reduction state.", SURGERY MAR 1990, vol. 107, no. 3, March 1990 (1990-03), pages 289-294, XP008164803, ISSN: 0039-6060**
• **DATABASE WPI Week 199017 Thomson Scientific, London, GB; AN 1990-130797 XP002712786, & SU 1 492 278 A (IVAN MOTHER AND CHILD RE) 7 July 1989 (1989-07-07)**
• **DATABASE WPI Week 198616 Thomson Scientific, London, GB; AN 1986-105767 XP002712787, & SU 1 183 073 A (KHARK CHILD HEALTH) 7 October 1985 (1985-10-07)**

**(Cont. next page)**

EP 2 579 037 B1

- JUNJI CHIDA ET AL: "Blood Lactate/ATP Ratio, as an Alarm Index and Real-Time Biomarker in Critical Illness", PLOS ONE, vol. 8, no. 4, 5 April 2013 (2013-04-05), page e60561, XP055079023, DOI: 10.1371/journal.pone.0060561
- Anonymous: "Reference range", Wikipedia, 14 April 2010 (2010-04-14), XP055118608, Retrieved from the Internet: URL:http://en.wikipedia.org/w/index.php?ti tle=Reference_ range&oldid=355903135 [retrieved on 2014-05-19]
- RIVERS EMANUEL ET AL: "Early goal-directed therapy in the treatment of severe sepsis and septic shock", NEW ENGLAND JOURNAL OF MEDICINE, MASSACHUSETTS MEDICAL SOCIETY, BOSTON, MA, US, vol. 345, no. 19, 8 November 2001 (2001-11-08), pages 1368-1377, XP007915321, ISSN: 1533-4406
- ROSARIO PEDRO WESLLEY: "Normal values of serum IGF-1 in adults: results from a Brazilian population.", ARQUIVOS BRASILEIROS DE ENDOCRINOLOGIA E METABOLOGIA 2010, vol. 54, no. 5, 2010, pages 477-481, ISSN: 1677-9487

## Description

### Technical Field

The present invention relates to a method for testing the severity of an illness by measuring adenosine triphosphate (hereinafter, also referred to as "ATP") level and the level of intermediate metabolite lactic acid of energy metabolism in a sample to calculate a lactic acid level / ATP level ratio, used as an index for the severity of the illness.

### Background Art

ATP is a chemical (nucleotide) that is used as energy required for all living organisms. For this reason, ATP assay has heretofore been used routinely for the purpose of determining the presence or absence of microbes contained in biological samples. Since ATP is mainly produced in intracellular organelle mitochondria, ATP has been assayed so far in order to determine mitochondrial functions. The conventional ATP assay techniques, however, have failed to efficiently extract ATP contained in biological samples. Due to such inaccurate information on the ATP concentrations in the biological samples, it has been difficult to accurately deduce the "state of intracellular energy required for living organisms" from measured ATP levels by use of this numeric value.

The present inventors have established a revolutionary technique of very effectively extracting ATP from a sample and accurately measuring the ATP concentration in the cell or the tissue (patent document 1: WO2009/096429; Method for extraction of nucleotide). This method offers accurate information on ATP concentrations in biological samples.

Meanwhile, the APACHE II scoring system (non-patent document 1: Knaus WA, Draper EA, Wagner DP, and Zimmerman JE: A severity of disease classification system. Critical Care Medicine 13: 818-829, 1985) for severity in hospitalized patients has been adopted since its revision in 1985 down to this day as a conventional technique of determining the "severity of an illness". Since the APACHE II score does not reflect real-time scores, risk markers or evaluation methods have been demanded as a substitute for the score. The APACHE II scoring system involves acute physiological scores consisting of 12 variables: 1) deep body temperature, 2) mean arterial pressure, 3) heart rate, 4) respiratory rate, 5) oxygenation, 6) arterial pH, 7) serum Na concentration, 8) serum K concentration, 9) serum creatinine concentration, 10) hematocrit value, 11) leucocyte count, and 12) the Glasgow coma score, and conducts evaluation based on the total of these scores plus age point and chronic health problem point. Alternatively, the SOFA scoring system (non-patent document 2: Vincent JL, Moreno R, Takala J, Willatts S, De Mendonca A, Bruining H, Reinhart CK, Suter PM, and Thijs LG: The SOFA (Sepsis-related Organ Failure Assessment) score to describe organ dysfunction/failure. On behalf of the Working Group on Sepsis-Related Problems of the European Society of Intensive Care Medicine. Intensive Care Medicine 7: 707-710, 1996) has been proposed to determine the degree of failure in important organs. The SOFA score is calculated from the total of acute physiological scores: 1) respiratory function, 2) platelet count, 3) bilirubin level, 4) blood pressure, 5) the Glasgow Coma Scale, and 6) serum creatinine concentration or daily urine output. Unfortunately, these APACHE II and SOFA scores do not serve as real-time markers due to many variables to be measured and time-consuming procedures for rounding up evaluation results as in the measurement of daily urine output. Thus, the establishment of evaluation methods has been demanded as a substitute for these systems.

### Prior Art Documents

#### Patent Document

Patent Document 1: International Publication No. WO2009/096429

#### Non-patent Documents

Non-patent Document 1: Critical Care Medicine 13: 818-829, 1985
Non-patent Document 2: Intensive Care Medicine 7: 707-710, 1996

### Summary of the Invention

### Object to be Solved by the Invention

An object of the present invention is directed to a method for evaluating the severity of an illness and is to provide a novel biomarker that is capable of assessing the severity of an illness in real time, unlike physiological scores such as the APACHE II or SOFA score, which quantifies the degree of organ failure, and to provide criteria for the assessment.

**Means to Solve the Object**

As a result of conducting diligent studies to attain the object, the present inventors have found that an established testing method using the method for extraction of nucleotide (patent document 1) by the present inventors can accurately measure an ATP level in a sample, thereby accurately deducing the "state of intracellular energy required for living organisms" from the ATP level, and by extension, determining the severity of an illness, and have further found that the severity of an illness can be determined in real time by evaluation with the level of lactic acid or ketone body that accumulates in blood due to the breakdown of *in vivo* energy metabolism accompanied by increased severity, and an ATP concentration in blood as an index (specifically, on the basis of a lactic acid concentration (mM)/ATP concentration (mM) ratio and/or a ketone body concentration (mM)/ATP concentration (mM) ratio). On the basis of these findings, the present invention has been completed.

Specifically, the present invention provides: [1] a method for testing the severity of an illness, that can determine the severity of an illness in real time, the method comprising the following steps A) to-D): A) a step of measuring adenosine triphosphate level in a sample; B) a step of measuring lactic acid level in the sample; C) a step of calculating lactic acid level / adenosine triphosphate level which is a ratio of the lactic acid level measured in step B to the adenosine triphosphate level measured in step A; and D) a step of using the ratio calculated in step C as an index for the severity of the illness;

[2] the testing method according to [1], wherein measuring the level of adenosine triphosphate in a sample comprises: 1) a step of treating the sample with a solution comprising a phenol compound and extracting adenosine triphosphate in the sample to measure the level of adenosine triphosphate contained in the sample; and 2) a step of measuring the level of the extracted adenosine triphosphate using a reagent for adenosine triphosphate assay; [3] the testing method according to [2], wherein the solution comprising a phenol compound has a pH of 4 to 10; [4] the testing method according to [2] or [3], wherein the solution comprising a phenol compound further comprises a protein denaturant; [5] the testing method according to any one of [2] to [4], wherein the phenol compound is phenol;

The present invention also provides: [6] the testing method according to any one of [1] to [5], wherein the sample is blood obtained from a test subject, and is assessed as abnormal when the ratio of the lactic acid level to the adenosine triphosphate level in the blood is higher than the upper limit 3.7 of its normal value; [7] the testing method according to [6], wherein the severity of an illness is assessed as mildly abnormal when the ratio of the lactic acid level to the adenosine triphosphate level in the blood is higher than the upper limit 3.7 of its normal value and equal to or lower than 8.0, and as severely abnormal when the ratio is higher than 8.0 and equal to or lower than 25.0, and as severely abnormal leading to death when a high value exceeding 25.0 continues for 6 hours or longer;

The present invention further provides:

[8] the testing method according to any one of [1] to [7], further comprising measuring a ketone body level in the sample;

[9] the testing method according to [8], wherein ketone body level / adenosine triphosphate level which is a ratio of the ketone body level to the adenosine triphosphate level in the sample is used as an index for the severity of an illness;

[10] the testing method according to [8] or [9], wherein the sample is blood obtained from a test subject, and is assessed as abnormal when the ratio of the ketone body level to the adenosine triphosphate level in the blood is higher than the upper limit 0.25 of its normal value.

**Effect of the Invention**

The testing method of the present disclosure can accurately deduce the "state of intracellular energy required for living organisms" from ATP levels, thereby evaluating or determining the severity of an illness. Alternatively, for example, from the breakdown of energy metabolism in which carbohydrates, lipids, and amino acids consumed as energy sources by every living thing cannot be exploited smoothly in ATP production or from the breakdown of metabolism in which ATP production cannot compensate for excessive energy consumption, the concentration of an intermediate metabolite lactic acid or ketone body of energy metabolism that accumulates *in vivo* can be associated with the ATP concentration, thereby evaluating or determining the severity of an illness. The testing method of the present invention has enabled the "severity of an illness" to be evaluated or determined as mild abnormality, severe abnormality, an exceedingly high mortality risk, or the like, which have not been expected so far from information on their respective measured values alone.

**Brief Description of Drawings**

[Figure 1] Figure 1 is a diagram showing the distributions of ATP levels in healthy individual-derived samples, ages, and sexes. (Reference Example 1)

[Figure 2] Figure 2 is a diagram showing the distributions of lactic acid levels in healthy individual-derived samples, ages, and sexes. (Reference Example 2)

[Figure 3] Figure 3 is a diagram showing the distributions of A-LES values calculated from ATP levels and lactic acid levels in healthy individual-derived samples, and the ages and sexes of the healthy individuals. (Reference Example 3)

[Figure 4A] Figure 4A is a diagram showing time-dependent changes in the severity of various patients in an intensive care unit monitored with ATP values in samples as an index. (Example 1)

[Figure 4B] Figure 4B is a diagram showing time-dependent changes in the severity of various patients in an intensive care unit monitored with A-LES (lactic acid level/ATP level) values as an index. (Example 1)

[Figure 5] Figure 5 is a diagram showing the comparison of movements in APACHE II scores and A-LES values between at the time of admission and at the time of discharge (or at the time of death) of patients managed in an intensive care unit. (Example 2)

[Figure 6] Figure 6 is a diagram showing ATP levels, lactic acid levels, A-LES values, ketone body (3-hydroxybutyric acid) levels, and A-KES values in the blood of healthy mice and severely ill mice with influenza infection. (Example 3)

**Mode of Carrying Out the Invention**

The method for testing the severity of an illness according to the present disclosure is not particularly limited as long as the method comprises measuring an ATP level in a sample. Alternatively, the testing method of the present disclosure may further comprise measuring the level of an intermediate metabolite of energy metabolism, such as lactic acid or ketone body, wherein the ratio of the level of the intermediate metabolite of energy metabolism to the ATP level is used in evaluation or determination. In the present disclosure, the "severity of an illness" refers to how grave the illness is at the time of ATP level testing, and is used herein interchangeably with the "degree of increased severity of the illness", the "degree of increased severity", etc. The severity of an illness can be figured out to determine the condition of the illness at the time of testing, thereby using the results as an index for the prediction of future course of the illness or using the results to decide a therapeutic strategy such as the selection of a treatment method. Specifically, when the severity of an illness is mild abnormality, therapeutic effects are observed or the future remission of the illness is expected. Alternatively, when the severity of an illness is severe abnormality, therapeutic effects are not observed or the future exacerbation of the illness is expected, also suggesting a possibility of leading to death at the worst.

Examples of the illness in the "severity of an illness" can include, but not particularly limited to, respiratory, vascular, cardiovascular, gastrointestinal, cranial nerve, kidney and urinary tract, endocrine, sensory organ, genital, and musculoskeletal diseases, multiple organ failure, and infectious diseases. These may be endogenous diseases such as genetic or lifestyle-related diseases or may be caused by pathogens or viral infections. Preferable examples thereof can include coronary artery disease, infective endocarditis, sepsis, pulmonary embolism, fulminant hepatitis C, influenza pneumonia, and influenza infection.

In the present invention, the sample is not particularly limited as long as it is a tissue or a body fluid collected from an organism. Preferable examples thereof can include blood samples derived from humans (test subjects) and non-human vertebrates (test animals) that may be to be tested in the present invention. Examples of the test animals can include mammals, fish, amphibians, reptiles, and birds. The test animals are preferably mammals such as monkeys, horses, cattle, sheep, goats, pigs, dogs, cats, rabbits, rats, and mice. Particularly, mice can be used preferably. The blood sample may be derived from any of arterial (A) blood, pulmonary arterial (PA) blood, venous (V) blood, central venous (CV) blood, and peripheral venous blood. A method for measuring an ATP level as shown below was able to confirm that ATP levels did not vary in the blood of one test subject, depending on sites from which the blood was collected (see Table 3). Also, an anticoagulant or an antiseptic or the like may be added appropriately or particular components in blood may be removed or concentrated to prepare the blood sample, without impairing the measurement of ATP levels and lactic acid or ketone body levels.

In the testing method of the present invention, the method for measuring an ATP level in a sample is not particularly limited and is preferably the measurement method described in patent document 1 because this method achieves accurate measurement. Particularly, it is preferred to treat the sample with a solution comprising a phenol compound immediately after blood collection, extract ATP from the sample, and measure the level of the extracted ATP.

Specifically, the measurement method comprises the following steps:

1) treating the sample with a solution comprising a phenol compound and extracting ATP from the sample in order to measure the level of ATP contained in the sample; and

2) measuring the level of the extracted ATP using a reagent for ATP assay.

In the testing method of the present invention, the phenol compound used in the step of extracting ATP contained in the sample is not particularly limited as long as the compound has a phenol group and is capable of extracting ATP from the sample. Phenol is particularly preferable. Also, the solution comprising a phenol compound may further comprise a protein denaturant. Any protein denaturant known in the art can be used as the protein denaturant. Particularly preferable examples thereof can include protein denaturants that may be used in conventional ATP extraction methods, for example, guanidine isocyanate, perchloric acid, TCA, and protein kinase K. In the case of a sample rich in proteins other than ATPase, the addition of the protein denaturant may denature and aggregate these proteins. In such a case, analyte nucleic acids such as ATP may get lost in the proteins thus aggregated by denaturation, making extraction difficult. This may interfere with accurate assay. Also, heat treatment used in the conventional ATP extraction methods may be used for sample treatment in combination with the method for extraction of nucleotide according to the present disclosure. In the case of a sample rich in proteins, however, nucleic acids may be incorporated in the proteins denatured by heating, as described above. Thus, in some cases, ATP can be extracted most efficiently by treatment with the solution comprising a phenol compound that is not combined with a protein denaturant or heat treatment.

The pH of the solution comprising a phenol compound is not particularly limited and is preferably set, for the most sensitive assay, to a pH at which a nucleic acid assay reagent such as a luciferase assay reagent can react most efficiently. In the case of sample treatment with the solution comprising a phenol compound, ATP is extracted more effectively than the conventional extraction methods even by treatment with a solution of any pH, for example, pH 4 to 10. For example, in ATP assay using a luciferase reagent, effective assay can be achieved around pH 7 to 9 according to the characteristics of luciferase. Accordingly, the pH of the solution comprising a phenol compound is preferably set to around 7 to 9, more preferably around 8. The solution comprising a phenol compound used in the step of extracting ATP can be used as a reagent for ATP extraction from the sample.

The solvent for the phenol compound is not particularly limited, and, for example, TE (10 mM Tris-HCl, pH 8.0, and 1 mM EDTA) can be used. A stabilizer may be further added thereto, if necessary. In the testing method of the present invention, the ATP level can be measured by a method appropriately selected according to an analyte of interest. Such a method can encompass methods known in the art as well as every measurement method that will be developed. The methods known in the art that can be applied to the present invention may be, for example, a method using luciferin-luciferase luminescence reaction or a method using ATP exchange reaction. Examples of the method for assaying ATP using luciferin-luciferase luminescence reaction include methods which involve contacting a luminescent reagent comprising luciferin and luciferase with target ATP in the presence of metal ions (e.g., magnesium ions) and measuring the intensity of the generated light. ATP can be assayed specifically and accurately using, for example, XL-ATP kit (manufactured by APRO Life Science Institute, Inc.) as a commercially available kit for ATP assay.

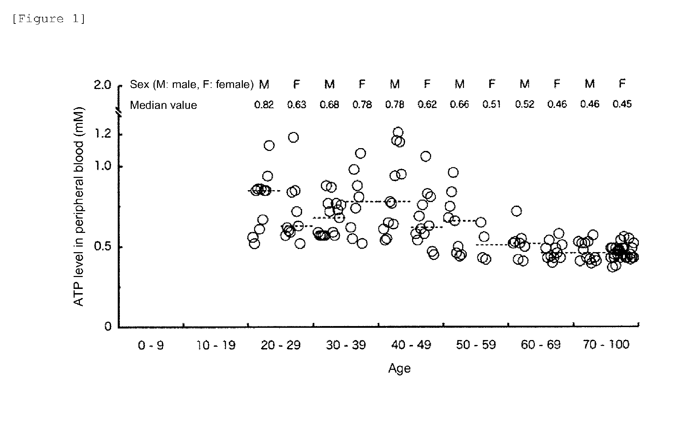

For establishing the method for testing the severity of an illness according to the present disclosure, it is required to figure out ATP levels in healthy individual-derived samples. Thus, peripheral venous blood was collected from each healthy volunteer, and an ATP level in the blood was measured by the method for assaying an ATP level. As a result, ATP was found at a concentration of 0.52 to 1.3 mM in most of the healthy individuals. Also, the level of ATP in blood was confirmed not to significantly differ depending on sex (see Figure 1). In the present invention, the normal value of the ATP level in the sample can be defined as 0.52 to 1.3 mM, more specifically 0.52 to 1.21 mM, with 0.72 mM as a median value. Also, the lower limit of the normal value can be defined as 0.52 mM.

In the method for testing the severity of an illness according to the present disclosure, specifically, the severity can be evaluated from the measured ATP level in the sample as follows: in a human, the severity can be assessed as having abnormality when the ATP level in the sample is lower than the lower limit 0.52 mM of its normal value. Furthermore, the severity of an illness in a human can be assessed as (1) mild abnormality or (2) severe abnormality according to the measured ATP level when the ATP level is lower than the lower limit 0.52 mM of its normal value.

The relationship between the ATP level and the severity of an illness in a human can be assessed according to the following criteria:

(1) mild abnormality when the ATP level is lower than 0.52 mM and equal to or higher than 0.3 mM; and

(2) severe abnormality when the ATP level is lower than 0.3 mM. The severity of an illness is assessed as a high mortality risk when the ATP level does not recover to 0.3 mM or higher within 6 to 24 hours, particularly within 24 hours.

The severity of an illness can be tested more effectively through energy metabolism by further measuring the level of an intermediate metabolite lactic acid or ketone body of energy metabolism in the sample. In the present invention, the "ketone body" refers to acetoacetic acid and 3-hydroxybutyric acid and excludes acetone. Thus, the ketone body level according to the present invention refers to the levels of acetoacetic acid and/or 3-hydroxybutyric acid. The level of 3-hydroxybutyric acid is often used as an index for the ketone body level because it can be measured within a few minutes using a high-speed measurement apparatus.

The lactic acid level or the ketone body level in the sample may be measured by a method known in the art or a method that will be developed as long as the method is capable of accurately measuring the lactic acid level or the ketone body level in blood. For example, a fully automatic blood gas analyzer (Bayer 860COT; available from Bayer HealthCare AG) or a simple analyzer (Lactate Pro; manufactured by ARKRAY, Inc.) can be used in the measurement of the lactic acid level. For example, "Total Ketone Body Kainos" or "Keto-Diastix (manufactured by Siemens Healthcare Diagnostics K.K.)" can be used in the measurement of the ketone body level. The assay can be conducted according to the measurement method recommended by each manufacturer.

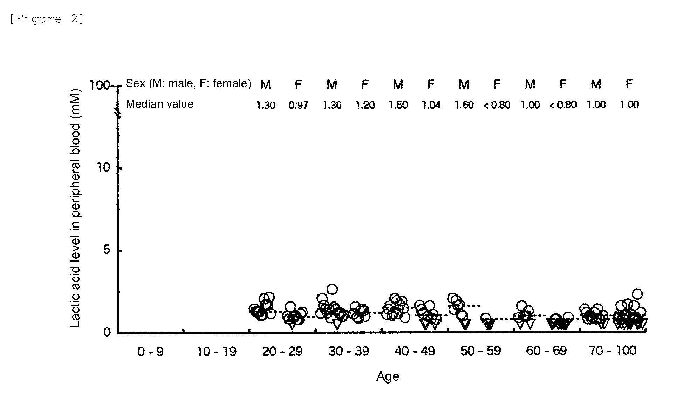

For establishing the method for testing the severity of an illness according to the present invention, it is preferred to figure out the levels of the intermediate metabolite lactic acid of energy metabolism in healthy individual-derived samples. Thus, peripheral venous blood was collected from each healthy volunteer, and a lactic acid level in the blood was measured by the method for measuring a lactic acid level. As a result, the level was shown to be equal to or lower than 2.7 mM in males and equal to or lower than 1.65 mM in females, demonstrating its concentration of equal to or lower than 2.7 mM in both males and females (see Figure 2). The median value of each age is shown in the diagram. In this context, levels of 0.8 mM or lower are indicated by the inverted triangle as equal to or lower than the detection limit of the instrument used (Lactate Pro; manufactured by ARKRAY, Inc.) and were described and calculated as 0.8 mM.

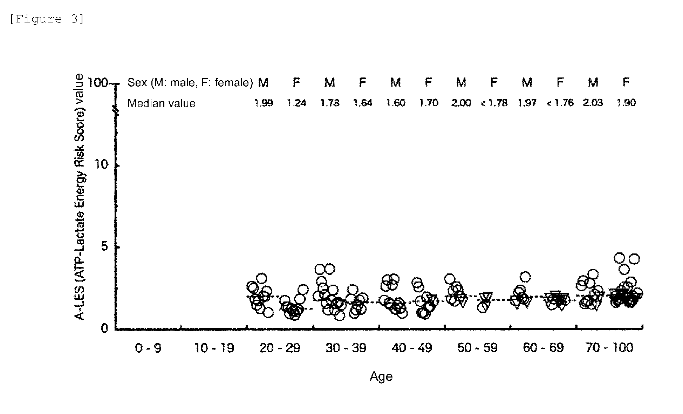

The ratio (A-LES value) of the lactic acid level to the ATP level measured by the method described above can be calculated, thereby testing the severity of an illness more accurately. The A-LES value can be calculated according to the formula I shown below. The value thus obtained by calculation can be used as a biomarker for the severity of an illness based on energy metabolism (see Figure 3).

```
(Formula I)

A-LES value = Lactic acid level (L; mM)/ATP level (A; mM)
```

The severity of an illness can be determined in more detail and more accurately on the basis of the A-LES value than the ATP level. According to the A-LES value, the severity of an illness can be assessed as (1) mild abnormality, (2) severe abnormality, or (3) severe abnormality leading to death.

The relationship between the A-LES value and severity in a human can be assessed according to the following criteria:

(1) mild abnormality when the A-LES value is higher than the upper limit 3.7 of its normal value and equal to or lower than 8.0;
(2) severe abnormality when the A-LES value is higher than 8.0 and equal to or lower than 25.0; and
(3) severe abnormality leading to death when a high value exceeding 25.0 continues for 6 to 24 hours or longer, particularly for 24 hours or longer. As for test animals, the relationship between the A-LES value and severity can be assessed by appropriately setting criteria according to their types.

The severity of an illness according to the present invention can be tested more effectively through energy metabolism by further measuring the level of the intermediate metabolite ketone body of energy metabolism in the sample.

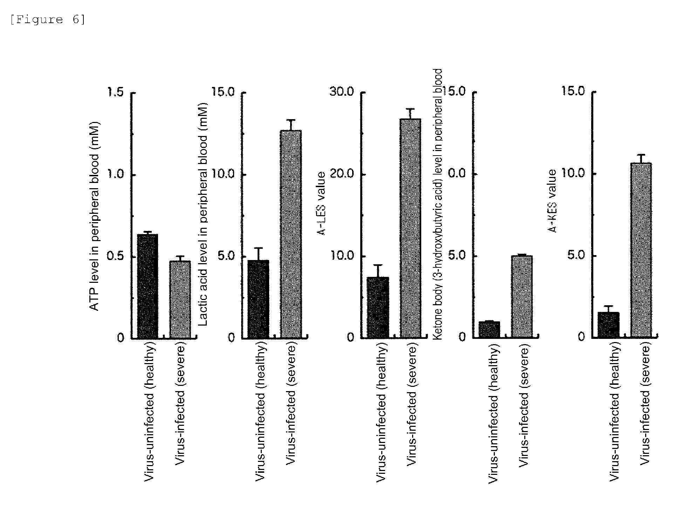

The ratio (A-KES value) of the ketone body level to the ATP level measured by the method described above can be calculated, thereby testing the severity of an illness more accurately. The A-KES value can be calculated according to the formula II shown below. The level of acetoacetic acid and/or 3-hydroxybutyric acid can be used as the ketone body level. The level of 3-hydroxybutyric acid is preferably used as the ketone body level because it can be measured within a few minutes using a high-speed measurement apparatus. The level of 3-hydroxybutyric acid is often used as the ketone body level. The value thus obtained by calculation can be used as a biomarker for the severity of an illness based on energy metabolism (see Figure 6).

```
(Formula II)

A-KES value = Ketone body level (K; mM)/ATP level (A; mM)
```

1 The severity of an illness can be determined more accurately on the basis of the A-KES value than the ATP level. The normal value of the ketone body level (total sum of acetoacetic acid and 3-hydroxybutyric acid levels) is considered as 130 μmol/L (0.13 mM) or lower (see Harumi Nishigaya et al., Japanese Journal of Medical Technology, 45, 3, 353 (1996); and Yutaka Haranou et al., Japanese Journal of Clinical Medicine, 48-suppl., 323 to 333 (1990)). The ATP level in blood is considered abnormal levels when exhibiting lower than 0.52 mM. Accordingly, in the case of using the total sum of acetoacetic acid and 3-hydroxybutyric acid levels as the ketone body level, an A-KES value equal to or higher than 0.13 mM/0.52 mM = 0.25 can be regarded abnormal. Thus, the severity of an illness in a human can be assessed with the upper limit 0.25 of the normal value of the A-KES value (total sum of acetoacetic acid and 3-hydroxybutyric acid levels /ATP level) as an index. Likewise, in the case of using the 3-hydroxybutyric acid level as the ketone body level, the severity of an illness in a human can also be assessed by setting the upper limit of its normal value. The relationship between the A-KES value and severity can be assessed by appropriately setting criteria according to the respective types of test subjects and test animals.

**Examples**

In order to help understand the present invention, the present invention will be described specifically with reference to Reference Examples and Examples shown below. However, the present invention is not limited to these examples by any means.

(Reference Example 1) Study on the distributions of ATP levels in healthy individual-derived samples, ages, and sexes

In this Reference Example, ATP levels and lactic acid levels in the peripheral venous blood of 142 healthy volunteers in total consisting of 68 males and 74 females in their 20s to 90s were measured for the purpose of figuring out ATP levels in healthy individual-derived samples to establish the method for testing the severity of an illness according to the present invention.

The ATP levels were measured after ATP extraction from the samples using XL-ATP kit (manufactured by APRO Life Science Institute, Inc.) according to the instruction manual. The reagent for ATP extraction used was a mixture of extraction reagent A (TE-saturated phenol, component: containing 69% phenol, pH 8.0) and extraction reagent B (chloroform, component: containing 99% chloroform) included in the kit and sterile ultrapure water at a ratio of 3:5:5 respectively. Specifically, 0.1 ml of the blood collected from each subject was added and mixed into the ATP extraction reagent (0.3 ml of extraction reagent A, 0.5 ml of extraction reagent B, and 0.5 ml of sterile ultrapure water). Organic solvent and aqueous layers were separated by centrifugation or the like, and the supernatant (aqueous layer) was then collected to extract ATP from the blood.

The assay results are shown in Figure 1 and Tables 1 and 2. The ATP levels in the peripheral venous blood hardly differed statistically significantly between males and females and exhibited the tendency to converge to the low value 0.5 mM in the older individuals. The median value was shown to be 0.82 mM in the males in their 20s, 0.63 mM in the females in their 20s, 0.68 mM in the males in their 30s, 0.78 mM in the females in their 30s, 0.78 mM in the males in their 40s, 0.62 mM in the females in their 40s, 0.66 mM in the males in their 50s, 0.51 mM in the females in their 50s, 0.52 mM in the males in their 60s, 0.46 mM in the females in their 60s, 0.46 mM in the males equal to or older than 70 years old, and 0.45 mM in the females equal to or older than 70 years old.

[Table 1]

| Healthy individual ID | Age/Sex | Blood collection site | Lactic acid level (mM) | ATP level (mM) | A-LES |
|---|---|---|---|---|---|
| H20M 01. | 20s/M | V | 1.49 | 0.56 | 2.61 |
| H20M 02. | 20s/M | V | 1.30 | 0.52 | 2.50 |
| H20M 03. | 20s/M | V | 1.29 | 0.85 | 1.84 |
| H20M 04. | 20s/M | V | 1.27 | 0.86 | 1.48 |
| H20M 05. | 20s/M | V | 1.06 | 0.61 | 1.74 |
| H20M 06. | 20s/M | V | 1.09 | 0.86 | 1.27 |
| H20M 07. | 20s/M | V | 2.07 | 0.67 | 3.09 |
| H20M 08. | 20s/M | V | 1.73 | 0.85 | 2.04 |
| H20M 09. | 20s/M | V | 1.69 | 0.85 | 1.99 |
| H20M 10. | 20s/M | V | 2.17 | 0.94 | 2.31 |
| H20M 11. | 20s/M | V | 1.17 | 1.13 | 1.04 |
| H20F 01. | 20s/F | V | 1.00 | 0.57 | 1.75 |
| H20F 02. | 20s/F | V | 0.84 | 0.62 | 1.36 |
| H20F 03. | 20s/F | V | 1.58 | 0.60 | 0.95 |
| H20F 04. | 20s/F | V | 0.74 | 0.59 | 1.35 |

(continued)

| Healthy individual ID | Age/Sex | Blood collection site | Lactic acid level (mM) | ATP level (mM) | A-LES |
|---|---|---|---|---|---|
| H20F 05. | 20s/F | V | 0.94 | 0.84 | 1.12 |
| H20F 06. | 20s/F | V | 1.03 | 1.18 | 0.87 |
| H20F 07. | 20s/F | V | 0.83 | 0.85 | 1.09 |
| H20F 08. | 20s/F | V | 0.88 | 0.72 | 1.22 |
| H20F 09. | 20s/F | V | 1.16 | 0.63 | 1.84 |
| H20F 10. | 20s/F | V | 1.26 | 0.52 | 2.42 |
| H30M 01. | 30s/M | V | 1.19 | 0.59 | 2.02 |
| H30M 02. | 30s/M | V | 2.06 | 0.57 | 3.65 |
| H30M 03. | 30s/M | V | 1.66 | 0.57 | 2.91 |
| H30M 04. | 30s/M | V | 1.43 | 0.57 | 2.51 |
| H30M 05. | 30s/M | V | 1.20 | 0.57 | 2.11 |
| H30M 06. | 30s/M | V | 1.40 | 0.88 | 1.59 |
| H30M 07. | 30s/M | V | 0.91 | 0.77 | 1.18 |
| H30M 08. | 30s/M | V | 2.64 | 0.72 | 3.67 |
| H30M 09. | 30s/M | V | 1.55 | 0.87 | 1.78 |
| H30M 10. | 30s/M | V | 1.41 | 0.59 | 2.39 |
| H30M 11. | 30s/M | V | <0.80 | 0.57 | 1.18 |
| H30M 12. | 30s/M | V | 1.21 | 0.77 | 1.57 |
| H30M 13. | 30s/M | V | 1.19 | 0.73 | 1.63 |
| H30M 14. | 30s/M | V | 0.97 | 0.68 | 0.82 |
| H30M 15. | 30s/M | V | 10.2 | 0.76 | 1.47 |
| H30F 01. | 30s/F | V | 1.14 | 0.62 | 1.84 |
| H30F 02. | 30s/F | V | 1.59 | 0.66 | 2.41 |
| H30F 03. | 30s/F | V | 0.95 | 0.98 | 0.97 |
| H30F 04. | 30s/F | V | 0.88 | 0.74 | 1.19 |
| H30F 05. | 30s/F | V | 1.31 | 0.88 | 1.49 |
| H30F 06. | 30s/F | V | 1.44 | 0.81 | 1.78 |
| H30F 07. | 30s/F | V | 1.33 | 1.06 | 1.23 |
| H30F 08. | 30s/F | V | 0.99 | 0.52 | 1.90 |
| H40M 01. | 40s/M | V | 1.10 | 0.62 | 1.77 |
| H40M 02. | 40s/M | V | 1.42 | 0.54 | 2.62 |
| H40M 03. | 40s/M | V | 1.65 | 0.55 | 3.00 |
| H40M 04. | 40s/M | V | 1.04 | 0.65 | 1.60 |
| H40M 05. | 40s/M | V | 1.17 | 0.78 | 1.50 |
| H40M 06. | 40s/M | V | 2.08 | 0.77 | 2.70 |
| H40M 07. | 40s/M | V | 1.95 | 0.64 | 3.05 |
| H40M 08. | 40s/M | V | 1.16 | 0.94 | 1.23 |
| H40M 09. | 40s/M | V | 1.68 | 1.16 | 1.45 |
| H40M 10. | 40s/M | V | 1.92 | 1.21 | 1.59 |
| H40M 11. | 40s/M | V | 1.48 | 1.16 | 1.29 |
| H40M 12. | 40s/M | V | 0.92 | 0.95 | 0.97 |
| H40F 01. | 40s/F | V | 1.64 | 0.58 | 2.83 |
| H40F 02. | 40s/F | V | 1.39 | 0.54 | 2.57 |
| H40F 03. | 40s/F | V | 1.17 | 0.69 | 1.70 |
| H40F 04. | 40s/F | V | 0.61 | 0.61 | 1.00 |
| H40F 05. | 40s/F | V | 0.78 | 0.76 | 1.02 |
| H40F 06. | 40s/F | V |  | 0.58 |  |
| H40F 07. | 40s/F | V | 0.96 | 1.06 | 0.93 |

(continued)

| Healthy individual ID | Age/Sex | Blood collection site | Lactic acid level (mM) | ATP level (mM) | A-LES |
|---|---|---|---|---|---|
| H40F 08. | 40s/F | V | 1.63 | 0.83 | 1.96 |
| H40F 09. | 40s/F | V | 0.86 | 0.63 | 1.37 |
| H40F 07. | 40s/F | V | 1.10 | 0.81 | 1.35 |
| H40F 08. | 42/F | V | 0.80 | 0.47 | 1.70 |
| H40F 09. | 46/F | V | <0.80 | 0.45 | |

[Table 2]

| Healty individual D | Age/Sex | Blood collection site | Lactic acid level (mM) | ATP level (mM) | A-LES |
|---|---|---|---|---|---|
| H50M 01. | 50s/M | V | 2.08 | 0.66 | 3.06 |
| H50M 02. | 50s/M | V | 1.38 | 0.75 | 1.84 |
| H50M 03. | 50s/M | V | 1.95 | 0.84 | 2.32 |
| H50M 04. | 50s/M | V | 1.64 | 0.96 | 1.71 |
| H50M 05. | 51/M | V | <0.80 | 0.44 | |
| H50M 06. | 54/M | V | 1.70 | 0.66 | 2.58 |
| H50M 07. | 57/M | V | 1.00 | 0.50 | 2.00 |
| H50M 08. | 58/M | V | <0.80 | 0.45 | |
| H50M 09. | 59/M | V | 1.10 | 0.46 | 2.39 |
| H50F 01. | 50s/F | V | 0.85 | 0.65 | 1.31 |
| H50F 02. | 51/F | V | <0.80 | 0.42 | |
| H50F 03. | 59/F | V | <0.80 | 0.43 | |
| H50F 04. | 59/F | V | <0.80 | 0.59 | |
| H60M 01. | 61/M | V | 1.60 | 0.72 | 2.22 |
| H60M 02. | 61/M | V | <0.80 | 0.50 | |
| H60M 03. | 62/M | V | 1. 00 | 0.55 | 1.82 |
| H60M 04. | 66/M | V | <0.80 | 0.53 | |
| H60M 05. | 68/M | V | 0.90 | 0.52 | 1.73 |
| H60M 06. | 68/M | V | 1.00 | 0.42 | 2.38 |
| H60M 07. | 68/M | V | 1.30 | 0.41 | 3.17 |
| H60M 08. | 69/M | V | 1.00 | 0.52 | 1.92 |
| H60F 01. | 62/F | V | 0.90 | 0.51 | 1.76 |
| H60F 02. | 63/F | V | 0.80 | 0.54 | 1.48 |
| H60F 03. | 63/F | V | <0.80 | 0.46 | |
| H60F 04. | 63/F | V | <0.80 | 0.58 | |
| H60F 05. | 65/F | V | <0.80 | 0.49 | |
| H60F 06. | 65/F | V | <0.80 | 0.43 | |
| H60F 07. | 65/F | V | 0.80 | 0.44 | 1.82 |
| H60F 08. | 65/F | V | <0.80 | 0.40 | |
| H60F 09. | 67/F | V | <0.80 | 0.49 | |
| H60F 10. | 69/F | V | <0.80 | 0.43 | |
| H60F 11. | 69/F | V | <0.80 | 0.43 | |
| H70M 01. | 70/M | V | 0.80 | 0.48 | 1.67 |
| H70M 02. | 73/M | V | 1.40 | 0.42 | 3.33 |
| H70M 03. | 74/M | V | 0.80 | 0.52 | 1.54 |
| H70M 04. | 76/M | V | 1.20 | 0.41 | 2.93 |
| H70M 05. | 76/M | V | 0.90 | 0.52 | 1.73 |
| H70M 06. | 77/M | V | 1.40 | 0.53 | 2.64 |
| H70M 07. | 78/M | V | 0.80 | 0.53 | 1.51 |

(continued)

| Healty individual D | Age/Sex | Blood collection site | Lactic acid level (mM) | ATP level (mM) | A-LES |
|---|---|---|---|---|---|
| H70M 08. | 79/M | V | 1.00 | | |
| H70M 09. | 79/M | V | 1.20 | 0.43 | 1.79 |
| H70F 01. | 71/F | V | 1.00 | 0.49 | 2.04 |
| H70F 02. | 71/F | V | <0.80 | 0.43 | |
| H70F 03. | 72/F | V | <0.90 | 0.49 | |
| H70F 04. | 73/F | V | 1.00 | 0.43 | 2.33 |
| H70F 05. | 73/F | V | 1.70 | 0.47 | 3.62 |
| H70F 06. | 73/F | V | 0.80 | 0.49 | 1.63 |
| H70F 07. | 73/F | V | 0.90 | 0.45 | 2.00 |
| H70F 08. | 73/F | V | 1.20 | 0.55 | 2.18 |
| H70F 09. | 74/F | V | <0.80 | 0.38 | |
| H70F 10. | 74/F | V | 1.00 | 0.49 | 2.04 |
| H70F 11. | 75/F | V | 0.80 | 0.45 | 1.78 |
| H70F 12. | 75/F | V | <0.80 | 0.47 | |
| H70F 13. | 75/F | V | 1.10 | 0.43 | 2.56 |
| H70F 14. | 75/F | V | 0.80 | 0.49 | 1.63 |
| H70F 15. | 75/F | V | <0.80 | 0.45 | |
| H70F 16. | 79/F | V | <0.80 | 0.43 | |
| H80M 01. | 80/M | V | 0.80 | 0.36 | 2.11 |
| H80M 02. | 81/M | V | <0.80 | 0.41 | |
| H80M 03. | 82/M | V | <0.80 | 0.57 | |
| H80M 04. | 86/M | V | 1.00 | 0.43 | 2.33 |
| H80F 01. | 80/F | V | 0.90 | 0.45 | 2.00 |
| H80F 02. | 80/F | V | 1.60 | 0.37 | 4.32 |
| H80F 03. | 81/F | V | 0.80 | 0.43 | 1.86 |
| H80F 04. | 81/F | V | 1.10 | 0.43 | 2.56 |
| H80F 05. | 83/F | V | 1.60 | 0.56 | 2.86 |
| H80F 06. | 84/F | | 0.80 | 0.49 | 1.63 |
| H80F 07. | 84/F | | 0.90 | 0.52 | 1.73 |
| H80F 08. | 84/F | | <0.80 | 0.43 | |
| H80F 09. | 84/F | | <0.80 | 0.42 | |
| H80F 10. | 84/F | | <0.80 | 0.48 | |
| H80F 11. | 87/F | V | <0.80 | 0.38 | |
| H90F 01. | 90/F | V | 2.30 | 0.54 | 4.20 |
| H90F 02. | 92/F | V | 0.80 | 0.45 | 1.78 |

(Reference Example 2) Study on the distributions of lactic acid levels in healthy individual-derived samples, ages, and sexes

In this Reference Example, lactic acid levels in the peripheral venous blood of 142 healthy volunteers in total shown in Reference Example 1 were measured for the purpose of figuring out lactic acid levels in healthy individual-derived samples. The lactic acid levels were measured using a fully automatic blood gas analyzer (Bayer 860COT; Bayer HealthCare AG) or a simple analyzer (Lactate Pro; ARKRAY, Inc.) according to the measurement method recommended by each manufacturer.

The distributions of the measured lactic acid levels (mM) in the healthy individual-derived samples shown in Tables 1 and 2, and the ages and sexes of the healthy individuals are shown in Figure 2. The lactic acid levels in the peripheral venous blood of 142 healthy volunteers in total did not exhibit the statistically significant difference among ages or between males and females. The median value was shown to be 1.30 mM in the males in their 20s, 0.97 mM in the females in their 20s, 1.30 mM in the males in their 30s, 1.20 mM in the females in their 30s, 1.50 mM in the males in their 40s, 1.04 mM in the females in their 40s, 1.60 mM in the males in their 50s, 0.85 mM in the females in their 50s, 1.00 mM in the males in their 60s, 0.80 mM in the females in their 60s, 1.00 mM in the males equal to or older than 70 years old, and 1.00 mM in the females equal to or older than 70 years old.

(Reference Example 3) Study on the distributions of A-LES values calculated from ATP levels and lactic acid levels in healthy individual-derived samples, and the ages and sexes of the healthy individuals

This Example was intended to figure out A-LES values in healthy individuals on the basis of the measured ATP levels and lactic acid levels in the healthy individual-derived samples obtained in Reference Examples 1 and 2.

The A-LES values were calculated according to the following formula I:

```
(Formula I)

A-LES value = Lactic acid level (L; mM)/ATP level (a; mM)
```

The distributions of the A-LES values in the healthy individual-derived samples shown in Tables 1 and 2, and the ages and sexes of the healthy individuals are shown in Figure 3. The A-LES values in 142 healthy volunteers in total consisting of 68 males and 74 females in their 20s to 90s did not exhibit the statistically significant difference among ages or between males and females. The median value was shown to be 1.99 in the males in their 20s, 1.24 in the females in their 20s, 1.78 in the males in their 30s, 1.64 in the females in their 30s, 1.60 in the males in their 40s, 1.70 in the females in their 40s, 2.00 in the males in their 50s, 1.78 or lower in the females in their 50s, 1.97 in the males in their 60s, 1.76 or lower in the females in their 60s, 2.03 in the males equal to or older than 70 years old, and 1.90 in the females equal to or older than 70 years old. In this context, the inverted triangle represents that the lactic acid level in the sample was equal to or lower than the detection limit (0.8 mM); thus the A-LES value was equal to or lower than this value.

(Reference Example 4) Study on the influence of difference in blood collection site on measured ATP levels in blood

This Reference Example was intended to confirm the absence of variations in blood samples differing in blood collection site. Blood was collected simultaneously from a plurality of sites in one hospitalized patient for the medical testing purpose (e.g., oxygen partial pressure and $CO_2$ partial pressure measurements). The respective ATP levels and lactic acid levels of these samples were measured, and the measured levels were examined by comparison. The ATP levels and the lactic acid levels were measured by the same approaches as in Reference Examples 1 and 3.

Table 3 shows ATP levels in blood samples collected from artery (A), pulmonary artery (PA), and central vein (CV), respectively. Either central venous (CV) blood or venous (V) blood may usually suffice without distinction. Thus, it was a conscious choice to collect blood only from the central vein (CV), not from the vein (V), in order to reduce the burdens on patients. As shown in the measurement results, the ATP levels in the blood samples collected from artery (A), pulmonary artery (PA), and central vein (CV) did not exhibit the significant difference thereamong. This demonstrated that an ATP level measured in a blood sample collected from any one blood collection site (i.e., artery (A), pulmonary artery (PA), central vein (CV), or vein (V)) sufficed for the determination of a "life-threatening state".

Table 3 also shows the lactic acid levels (mM) and A-LES values (ratio of the lactic acid level to the ATP level) in addition to the ATP levels (mM).

[Table 3]

| Patient ID | Disease name | Blood collection date | Blood collection site | Lactic acid level (mM) | ATP level (mM) | A-LES | APACHE II score |
|---|---|---|---|---|---|---|---|
| P 23. | Coronary artery disease | 0113 2010 0h | A | 1.47 | 0.53 | 2.77 | 23 |
| P 23. | | 0113 2010 0h | PA | 1.30 | 0.57 | 2.26 | 23 |
| P 23. | | 0113 2010 0h | CV | 1.37 | 0.53 | 2.58 | 23 |

(continued)

| Patient ID | Disease name | Blood collection date | Blood collection site | Lactic acid level (mM) | ATP level (mM) | A-LES | APACHE II score |
|---|---|---|---|---|---|---|---|
| P 27. | Coarctation of aorta, Coronary artery disease | 0201 2010 0h | A | 1.79 | 0.70 | 2.56 | 13 |
| P 27. | | 0201 2010 0h | PA | 1.69 | 0.72 | 2.35 | 13 |
| P 27. | | 0201 2010 0h | CV | 1.97 | 0.73 | 2.70 | 13 |
| P 27. | | 0201 2010 3h | A | 1.95 | 0.83 | 2.35 | 13 |
| P 27. | | 0201 2010 3h | PA | 1.97 | 0.83 | 2.37 | 13 |
| P 27. | | 0201 2010 3h | CV | 2.16 | 0.80 | 2.70 | 13 |
| P 27. | | 0202 2010 6h | A | 2.09 | 0.34 | 6.15 | 13 |
| P 27. | | 0202 2010 6h | PA | 2.01 | 0.35 | 5.74 | 13 |
| P 27. | | 0202 2010 6h | CV | 2.01 | 0.34 | 5.91 | 13 |
| P 27. | | 0202 2010 24h | A | 1.68 | 0.71 | 2.37 | 12 |
| P 27. | | 0202 2010 24h | PA | 1.69 | 0.71 | 2.38 | 12 |
| P 27. | | 0202 2010 24h | CV | 1.63 | 0.74 | 2.20 | 12 |
| P 28. | Acute myocardial infarction (AMI) | 0201 2010 0h | A | 2.88 | 0.96 | 3.00 | |
| P 28. | | 0201 2010 0h | PA | 2.88 | 0.99 | 2.91 | |
| P 28. | | 0201 2010 0h | CV | 2.76 | 0.98 | 2.82 | |
| P 28. | | 2010 0h | A | 2.47 | 0.55 | 4.49 | 13 |
| P 28. | | 2010 0h | PA | 2.49 | 0.53 | 4.70 | 13 |
| P 28. | | 2010 0h | CV | 2.60 | 0.55 | 4.73 | 13 |
| P 29. | Coronary artety disease | 0203 2010 0h | A | 2.29 | 0.36 | 6.36 | 9 |
| P 29. | | 0203 2010 0h | PA | 2.43 | 0.40 | 6.08 | 9 |
| P 29. | | 0203 2010 0h | CV | 2.73 | 0.37 | 7.38 | 9 |
| P 30. | Heart failure, Renal failure | 0205 2010 3h | A | 1.64 | 0.33 | 4.97 | 10 |
| P 30. | | 0205 2010 3h | CV | 1.51 | 0.34 | 4.44 | 10 |

(Example 1) ATP levels, lactic acid levels, and A-LES values in the blood of patients who were admitted into an intensive care unit

In this Example targeting 43 patients managed in an emergency intensive care unit, ATP levels and lactic acid levels in samples derived from collected venous peripheral blood were measured after approval of the ethics committee of the University of Tokushima, and the severity of each patient was evaluated on the basis of A-LES values calculated from the obtained results. The ATP levels and the lactic acid levels were measured by the same approaches as the methods described in Reference Examples 1 and 3.

The A-LES values and APACHE II scores as conventional criteria for the determination of severity are shown in Tables 4 to 8. Of 43 patients, 8 patients died during hospitalization in the emergency intensive care unit: P17, P18, P43, P49, P50, P54, P60, and P63.

[Table 4]

| Group A (Mild case → Remission) | | | | | | | |
|---|---|---|---|---|---|---|---|
| Patient ID | Disease name | Blood collection date (elapsed time) | Blood collection site | Lactic acid level (mM) | ATP level (mM) | A-LES | APACHE II score |
| P 01. | Infective endocarditis, Cerebral infarction | Be fore | A | 1.26 | 0.69 | 1.83 | 13 |
| P 01. | | 1111 2009 0h | A | 1.52 | 0.69 | 2.20 | 12 |
| P 01. | | 1111 2009 3h | A | 1.24 | 0.38 | 3.26 | |
| P 01. | | 1111 2009 6h | A | 2.09 | 0.42 | 4.96 | |
| P 01. | | 1112 2009 24h | A | 1.27 | 0.70 | 1.81 | 10 |
| P 01. | | 1113 2009 2d | A | 2.01 | 0.52 | 3.87 | 13 |
| P 01. | | 1115 2009 3d | A | 1.51 | 0.38 | 3.97 | 15 |
| P 01. | | 1115 2009 4d | A | 0.71 | 0.38 | 1.87 | 15 |
| P 01. | | 1116 2009 5d | A | 0.76 | 0.63 | 1.21 | 15 |
| P 02. | Cerebellar hemorrhage | 1112 2009 0h | A | 4.43 | 0.61 | 7.26 | 17 |
| P 02. | | 1112 2009 3h | A | 5.77 | 0.68 | 8.49 | |
| P 02. | | 1113 2009 24h | A | 1.63 | 0.60 | 2.72 | 15 |
| P 03. | Influenza pneumonia | 1111 2009 0h | A | 1.91 | 0.71 | 2.69 | 16 |
| P 03. | | 1112 2009 3h | A | 1.58 | 0.44 | 3.59 | |
| P 03. | | 1112 2009 6h | A | 3.31 | 0.43 | 7.70 | |
| P 03. | | 1113 2009 24h | A | 2.67 | 0.71 | 3.76 | 14 |
| P 03. | | 1114 2009 3d | A | 2.73 | 0.36 | 7.58 | 18 |
| P 03. | | 1115 2009 4d | A | 1.98 | 0.35 | 5.66 | 14 |
| P 03. | | 1116 2009 5d | A | 1.66 | 0.55 | 3.02 | 17 |
| P 07. | Left atrial myxoma | 1116 2009 0h | A | 5.67 | 0.81 | 7.00 | 6 |
| P 07. | | 1116 2009 3h | A | 6.54 | 0.45 | 14.53 | |
| P 07. | | 1117 2009 6h | A | 6.72 | 0.36 | 18.67 | |
| P 07. | | 1117 2009 24h | A | 1.59 | 0.32 | 5.26 | 12 |
| P 07. | | 1118 2009 3d | A | 3.74 | 0.67 | 5.58 | 8 |
| P 07. | | 1119 2009 5d | A | 1.12 | 0.51 | 1.84 | 9 |
| P 13. | Unstable angina | 1126 2009 0h | A | 0.96 | 0.37 | 2.65 | 9 |
| P 13. | | 1126 2009 0h | A | 1.44 | 0.41 | 3.51 | |
| P 13. | | 1127 2009 3h | A | 2.84 | 0.48 | 5.92 | |
| P 13. | | 1127 2009 6h | A | 2.46 | 0.33 | 7.45 | |
| P 13. | | 1127 2009 24h | A | 1.12 | 0.42 | 2.67 | 6 |
| P 16. | Influenza pneumonia (at time of tracheal cannulation) | 1215 2009 0h | A | 2.95 | 0.86 | 3.43 | 9 |
| P 16. | | 1216 2009 3h | A | 2.63 | 0.93 | 2.83 | |
| P 16. | | 1216 2009 6h | A | 2.56 | 0.92 | 2.78 | |
| P 16. | | 1217 2009 3d | A | 2.03 | 0.72 | 2.82 | 4 |
| P 16. | | 1218 2009 4d | A | 1.88 | 0.59 | 3.19 | 5 |
| P 16. | | 1219 2009 5d | A | 1.75 | 0.35 | 5.00 | 3 |
| P 16. | | 1220 2009 6d | A | 1.71 | 0.55 | 3.11 | 8 |
| P 16. | (initiation date of Relenza inhalation) | 0109 2010 26d | A | 2.27 | 0.74 | 3.07 | 18 |
| P 16. | After becoming negative for PCR (Sw-Flu) | 0119 2010 36d | A | 1.80 | 0.62 | 2.90 | 13 |
| P 17. | Interstitial pneumonia, MRSA empyema | 0107 2010 0h | A | 1.62 | 0.31 | 5.23 | 27 |
| P 17. | | 0109 2010 24h | A | 1.09 | 0.45 | 2.42 | 13 |

(continued)

| Group A (Mild case → Remission) | | | | | | | |
|---|---|---|---|---|---|---|---|
| Patient ID | Disease name | Blood collection date (elapsed time) | Blood collection site | Lactic acid level (mM) | ATP level (mM) | A-LES | APACHE II score |
| P 17. | (after steroid pulse) | 0110 2010 3d | A | 1.16 | 0.42 | 2.76 | 14 |
| P 17. | | 0121 2010 14d | A | 0.89 | 0.47 | 1.89 | 19 |
| P 17. | | 0126 2010 19d | A | 1.17 | 0.51 | 2.29 | 22 |
| P 26. | Esophagus cancer | 0118 2010 0h | A | 6.82 | 0.56 | 12.18 | 14 |
| P 26. | | 0118 2010 3h | A | 7.19 | 0.43 | 16.72 | |
| P 26. | | 0119 2010 6h | A | 4.77 | 0.39 | 12.23 | |
| P 26. | | 0119 2010 2d | A | 2.10 | 0.49 | 4.29 | 15 |
| P 26. | | 0120 2010 3d | A | 1.18 | 0.46 | 2.57 | 13 |
| P 26. | | 0122 2010 5d | A | 1.11 | 0.52 | 2.13 | 13 |

[Table 5]

| | | | | | | | | |
|---|---|---|---|---|---|---|---|---|
| P 27. | Coarctation of aorta, | 0201 2010 0h | A | | 1.79 | 0.70 | 2.56 | 13 |
| P 27. | Coronary artery disease | 0201 2010 3h | A | | 1.95 | 0.83 | 2.35 | |
| P 27. | | 0202 2010 6h | A | | 2.09 | 0.34 | 6.15 | |
| P 27. | | 0202 2010 24h | A | | 1.68 | 0.71 | 2.37 | 12 |
| P 27. | | 0203 2010 3d | A | | 1.27 | 0.64 | 1.98 | 13 |
| P 28. | Acute myocardial infarction (AMI) | 0201 2010 0h | A | | 2.88 | 0.96 | 3.00 | |
| P 28. | | 0202 2010 | A | | 6.43 | 0.57 | 11.28 | |
| P 28. | | 0203 2010 | A | | 2.51 | 0.51 | 5.12 | |
| P 28. | | 2010 0h | A | | 2.47 | 0.55 | 4.49 | 13 |
| P 28. | | 0204 2010 0h | A | | | 0.48 | | |
| P 28. | | 0204 2010 0h | A | | 2.13 | 0.52 | 4.10 | |
| P 28. | | 0205 2010 24h | A | | 1.49 | 1.24 | 1.20 | 8 |
| P 28. | (immediately after closing of chest) | 0210 2010 5d | A | | 1.45 | 0.96 | 1.51 | 13 |
| P 28 | (after removal of IABP) | 0215 2010 10d | A | | 1.47 | 0.88 | 1.57 | 10 |
| P 29. | Coronary artery disease | 0203 2010 0h | A | | 2.29 | 0.36 | 6.36 | 9 |
| P 29. | | 0203 2010 3h | A | | 1.24 | 0.67 | 1.85 | |
| P 30. | Heart failure, Renal failure | 0204 2010 0h | A | | 1.95 | 0.71 | 2.75 | 10 |
| P 30. | | 0205 2010 3h | A | | 1.64 | 0.33 | 4.97 | |
| P 30. | | 0205 2010 6h | A | | 1.16 | 0.41 | 2.83 | |
| P 30. | | 0205 2010 24h | A | | | 0.83 | | 11 |
| P 34. | Infective endocarditis) | 0301 2010 0h | A | | 3.38 | 0.77 | 4.39 | 8 |
| P 34. | | 0301 2010 3h | A | | 2.94 | 0.52 | 5.65 | |
| P 34. | | 0302 2010 6h | A | | 1.59 | 0.48 | 3.31 | |
| P 34. | | 0302 2010 24h | A | | 1.12 | 0.83 | 1.36 | 9 |
| P 37. | Left putaminal hemorrhage | 0422 2010 0h | A | | 2.06 | 0.40 | 5.15 | |
| P 37. | | 0423 2010 3h | A | | 2.29 | 0.34 | 6.74 | |
| P 37. | | 0423 2010 6h | A | | 2.22 | 0.51 | 4.34 | |

(continued)

| | | | | | | | |
|---|---|---|---|---|---|---|---|
| P 39. | Acute subdural hematoma | 0430 2010 0h | A | (immeasu rable) | 0.44 | | |
| P 39. | | 0430 2010 3h | A | 4.46 | 0.47 | 9.49 | |
| P 39. | | 0430 2010 6h | A | 2.82 | 0.42 | 6.71 | |
| P 39. | | 0502 2010 24h | A | 0.98 | 0.42 | 2.33 | |
| P 39. | | 0504 2010 3d | A | 0.85 | 0.24 | 3.54 | |
| P 39. | | 0505 2010 4d | A | 1.49 | 0.44 | 3.39 | |
| P 39. | | 0506 2010 5d | A | 2.18 | 0.52 | 4.19 | |
| P 44. | Cardiogenic pulmonary edema | 0503 2010 0h | A | 1.23 | 0.38 | 0.38 | 9 |
| P 44. | | 0504 2010 | A | 2.42 | 0.32 | 0.32 | 8 |
| P 44. | | 0505 2010 2d | A | 1.41 | 0.74 | 0.74 | 8 |
| P 44. | | 0506 2010 3d | A | 1.20 | 0.58 | 0.58 | 8 |
| P 57. | Cardiogenic pulmonary edema | 0909 2010 | A | 1.43 | 0.50 | 2.86 | 20 |
| P 57. | | 0910 2010 | A | 1.30 | 0.54 | 2.41 | 19 |
| P 57. | | 0912 2010 | A | 1. 06 | 0.51 | 2.08 | 16 |

[Table 6]

| Group B (Severe case → Remission) | | | | | | | |
|---|---|---|---|---|---|---|---|
| Patient ID | Disease name | Blood collection date(elapsed time) | Blood collection site | Lactic acid level (mM) | ATP level (mM) | A-LES | APACHE II score |
| P 08. | Abdominal aortic aneurysm | 1118 2009 0h | A | 2.45 | 0.19 | 12.89 | 13 |
| P 08. | | 1118 2009 3h | A | 1.84 | 0.74 | 2.49 | |
| P 08. | | 1118 2009 6h | A | 2.36 | 0.30 | 7.87 | |
| P 08. | | 1119 2009 24h | A | 1.16 | 0.78 | 1.49 | 16 |
| P 10. | Perforative peritonitis | 1122 2009 0h | A | 2.68 | 0.29 | 9.17 | 15 |
| P 10. | | 1122 2009 3h | A | 4.91 | 0.33 | 14.88 | |
| P 10. | | 1122 2009 6h | A | 3.06 | 0.30 | 10.20 | |
| P 10. | | 1123 2009 24h | A | 1.87 | 0.52 | 3. 60 | 12 |
| P 20. | Sepsis | 1128 2009 0h | V | 2.19 | 0.16 | 13.69 | 28 |
| P 20. | | 1128 2009 3h | V | 1.77 | 0.15 | 11.80 | |
| P 20. | | 1128 2009 6h | V | 1.70 | 0.20 | 8.50 | |
| P 20. | | 1129 2009 24h | V | 5.30 | 0.31 | 17.10 | 24 |
| P 20. | | 0101 2010 | V | 9.20 | 0.43 | 21.40 | 28 |
| P 20. | (after start of CHDF) | 0106 2010 9d | V | 2.27 | 0.56 | 4.05 | 19 |
| P 20. | (after removal of artificial respirator) | 0116 2010 19d | A | 1.91 | 0.48 | 3.90 | 17 |
| P 20. | | 0119 2010 22d | A | 2.45 | 0.31 | 7.90 | 20 |
| P 20. | | 0126 2010 29d | A | 1.45 | 0.63 | 2.30 | 15 |
| P 20. | | 0202 2010 36d | A | 1.21 | 0.65 | 1.86 | 12 |
| P 20. | | 0210 2010 44d | A | 2.35 | 0.77 | 3.05 | 19 |
| P 19. | After PD operation, Sepsis | 0113 2010 0h | A | 2.77 | 0.29 | 9.55 | 20 |
| P 19. | | 0113 2010 3h | A | | 0.27 | | |
| P 19. | | 0113 2010 6h | A | | 0.29 | | |
| P 19. | | 0113 2010 24h | A | 0.90 | 0.33 | 2.73 | 13 |
| P 25. | Esophagus cancer | 0118 2010 0h | A | 6.82 | 0.56 | 12.18 | 14 |
| P 25. | | 0118 2010 3h | A | 7.19 | 0.43 | 18.72 | |

(continued)

| Group B (Severe case → Remission) | | | | | | | |
|---|---|---|---|---|---|---|---|
| Patient ID | Disease name | Blood collection date(elapsed time) | Blood collection site | Lactic acid level (mM) | ATP level (mM) | A-LES | APACHE II score |
| P 25. | | 0119 2010 6h | A | 4.77 | 0.39 | 12.23 | |
| P 25. | | 0119 2010 2d | A | 2.10 | 0.49 | 4.29 | 15 |
| P 25. | | 0120 2010 3d | A | 1.18 | 0.46 | 2.57 | 13 |
| P 25. | | 0122 2010 5d | A | 1.11 | 0.52 | 2.13 | 13 |
| P 32. | Infective endocarditis | 0210 2010 0h | A | 7.11 | 0.48 | 14.81 | 5 |
| P 32. | | 0211 2010 5h | A | 3.01 | 0.47 | 6.40 | |
| P 32. | | 0211 2010 9h | | 1.42 | 0.41 | 3.46 | |
| P 32. | | 0212 2010 24h | A | 0.84 | 0.69 | 1.22 | 9 |
| P 35. | Acute myocardial infarction (AMI), Irregular heartbeat, CPA | 0302 2010 1d | A | 7.67 | 1.01 | 7.59 | 28 |
| P 35. | | 0303 2010 2d | A | 2.83 | 1.08 | 2.52 | 27 |
| P 35. | | 0304 2010 3d | | | 1.00 | | 28 |
| P 35. | | 0305 2010 4d | | 1.72 | 0.98 | 1.76 | 26 |
| P 36. | Unstable angina | 0419 2010 0h | A | 1.69 | 0.25 | 6.76 | |
| P 36. | | 0419 2010 3h | A | 2.64 | 0.34 | 7.76 | |
| P 36. | | 0419 2010 6h | A | 3.00 | 0.24 | 12.50 | |
| P 36. | | 0421 2010 24h | A | 2.77 | 0.46 | 6.02 | |
| P 36. | | 0423 2010 2d | A | 1.30 | 0.53 | 2.45 | |
| P 36. | | 0424 2010 3d | | 1.21 | 0.31 | 3.09 | |
| P 36. | | 0425 2010 4d | | 1.91 | 0.24 | 7.96 | |
| P 36. | | 0426 2010 5d | V | 1.35 | 0.30 | 4.50 | |
| P 36. | 2nd admission (cardiac tamponade) | 0502 2010 11d | A | 0.94 | 0.39 | 2.46 | |
| P 36. | | 0504 2010 13d | | 1.02 | 0.33 | 3.09 | |
| P 36. | | 0505 2010 14d | | 1.07 | 0.44 | 2.43 | |
| P 36. | | 0506 2010 15d | A | 1.27 | 0.47 | 2.70 | |
| P 41. | Acute subdural hemorrhage | 0430 2010 0h | A | (immeasurable) | 0.44 | | |
| P 41. | | 0430 2010 3h | A | 4.46 | 0.47 | 9.49 | |
| P 41. | | 0430 2010 6h | A | 2.82 | 0.42 | 6.71 | |
| P 41. | | 0502 2010 24h | A | 0.98 | 0.42 | 2.33 | |
| P 41. | | 0504 2010 3d | A | 0.85 | 0.24 | 3.54 | |
| P 41. | | 0505 2010 4d | A | 1.49 | 0.44 | 3.39 | |
| P 41. | | 0506 2010 5d | | 2.18 | 0.52 | 4.19 | |

[Table 7]

| | | | | | | | |
|---|---|---|---|---|---|---|---|
| P 42. | Heart failure/after ADS operation | 0430 2010 3h | | 1.53 | 0.23 | 7.09 | 20 |
| P 42. | | 0430 2010 6h | | 1.67 | 0.22 | 7.59 | 20 |
| P 42. | | 0501 2010 2d | A | 1.74 | 0.31 | 5.61 | 17 |
| P 42. | | 0502 2010 3d | A | 1.46 | 0.31 | 3.49 | 18 |

(continued)

| | | | | | | | |
|---|---|---|---|---|---|---|---|
| P 45. | Aortic insufficiency | 0510 2010 0h | A | 6.10 | 0.42 | 14.52 | 11 |
| P 45. | | 0510 2010 3h | A | 4.48 | 0.37 | 12.05 | 11 |
| P 45. | | 0510 2010 8h | A | 2.92 | 0.32 | 9.13 | 11 |
| P 45. | | 0511 2010 24h | A | 1.47 | 0.59 | 2.49 | 11 |
| P 47. | Dissecting aortic aneurysm/Mediastinitis | 0514 2010 0h | A | 14.82 | 0.36 | 41.17 | 20 |
| P 47. | | 0514 2010 6h | A | 9.98 | 0.45 | 22.18 | 20 |
| P 47. | | 0514 2010 12h | A | 5.65 | 0.41 | 13.78 | 20 |
| P 47. | | 0514 2010 18h | A | 2.54 | 0.51 | 5.18 | 20 |
| P 47. | | 0515 2010 24h | A | 2.43 | 0.54 | 4.50 | 19 |
| P 47. | | 0517 2010 3d | | 1.43 | 0.50 | 2.86 | 17 |
| P 47. | | 0518 2010 4d | | 1.46 | 0.47 | 3.11 | 18 |
| P 47. | | 0519 2010 5d | A | 1.57 | 0.34 | 4.62 | 13 |
| P 47. | | 0520 2010 6d | A | 1.13 | 0.25 | 4.52 | 17 |
| P 47. | | 0521 2010 7d | | 2.37 | 0.26 | 9.12 | 15 |
| P 47. | | 0522 2010 8d | | 1.40 | 0.39 | 3.59 | 19 |
| P 47. | | 0523 2010 9d | | 1.15 | 0.41 | 2.80 | 18 |
| P 47. | | 0524 2010 10d | | 1.39 | 0.40 | 3.45 | 17 |
| P 47. | | 0525 2010 11d | A | 1.05 | 0.37 | 2.84 | 16 |
| P 47. | | 0526 2010 12d | A | 1.09 | 0.28 | 3.89 | 16 |
| P 47. | | 0527 2010 13d | | 1.67 | 0.41 | 4. 07 | |
| P 52. | Septic shock | 0616 2010 | A | 4.10 | 0.34 | 12.06 | 41 |
| P 52. | | 0618 2010 | A | 5.52 | 0.35 | 15.77 | 23 |
| P 52. | | 0619 2010 | A | 2.23 | 0.16 | 13.94 | 24 |
| P 52. | Exploratory laparotomy | 0620 2010 | A | 1.89 | 0.17 | 11.12 | 30 |
| P 52. | | 0621 2010 | A | 1.80 | 0.23 | 7.83 | 23 |
| P 52. | | 0622 2010 | A | 1.19 | 0.46 | 2.59 | 30 |
| P 52. | | 0625 2010 | A | 0.89 | 0.48 | 1.85 | 13 |
| P 53. | Tricuspid regurgitation | 0712 2010 | A | 5.39 | 0.35 | 15.40 | 19 |
| P 53. | | 0713 2010 | A | 9.08 | 0.40 | 22.70 | 22 |
| P 53. | | 0714 2010 | A | 1.78 | 0.34 | 5.24 | 19 |
| P 53. | | 0715 2010 | A | 1.53 | 0.39 | 3.92 | 14 |
| P 53. | | 0716 2010 | A | 1.24 | 0.44 | 2.82 | 13 |
| P 56. | Septic shock (unknown focus) | 0809 2010 | A | 3.79 | 0.32 | 11.84 | 31 |
| P 56. | | 0810 2010 | A | 2.85 | 0.36 | 7.92 | 31 |
| P 56. | | 0811 2010 | A | 2.63 | 0.40 | 6.58 | 31 |
| P 56. | | 0812 2010 | A | 2.49 | 0.43 | 5.79 | 18 |
| P 56. | | 0818 2010 | A | 2.18 | 0.46 | 4.74 | 17 |
| P 56. | | 0819 2010 | A | 1.50 | 0.49 | 3.06 | 15 |
| P 58. | Pulmonary hemorrhage | 0920 2010 | A | 6.25 | 0.35 | 17.86 | 32 |
| P 58. | | 0921 2010 | A | 2.76 | 0.52 | 5.31 | 18 |
| P 58. | | 0922 2010 | A | 2.27 | 0.65 | 3.49 | 22 |
| P 58. | | 0923 2010 | A | 2.13 | 0.65 | 3.28 | 23 |
| P 59. | Aneurism of aortic arch | 0830 2010 | A | 9.20 | 0.57 | 16.14 | 12 |
| P 59. | | 0830 2010 | A | 8.58 | 0.58 | 14.80 | 12 |
| P 59. | | 0830 2010 | A | 8.21 | 0.61 | 13.46 | 12 |
| P 59. | | 0831 2010 | A | 8.04 | 0.67 | 12.00 | 8 |
| P 59. | | 0831 2010 | A | 6.52 | 0.68 | 9.59 | 8 |
| P 59. | | 0831 2010 | A | 2.48 | 0.68 | 3.65 | 8 |

(continued)

| | | | | | | | |
|---|---|---|---|---|---|---|---|
| P 59. | | 0903 2010 | A | 1.93 | 0.59 | 3.27 | 6 |
| P 62. | Sepsis | 0301 2011 | A | 7.26 | 0.29 | 25.03 | |
| P 62. | | 0301 2011 | A | 4.56 | 0.37 | 12.59 | |
| P 62. | | 0302 2011 | A | 4.43 | 0.47 | 9.43 | |
| P 62. | | 0303 2011 | A | 2.20 | 0.58 | 3.79 | |
| P 62. | | 0307 2011 | A | 2.43 | | | |

[Table 8]

| Group C (Severe case → Death) | | | | | | | |
|---|---|---|---|---|---|---|---|
| Patient ID | Disease name | Blood collection date(elapsed time) | Blood collection site | Lactic acid level (mM) | ATP level (mM) | A-LES | APACHE II score |
| P 17. | Pulmonary embolism | 1219 2009 0h | A | 7.29 | 0.29 | 25.14 | 25 |
| P 17. | | 1219 2009 3h | A | 10.60 | 0.18 | 66.25 | |
| P 17. | | 1219 2009 6h | A | 13.20 | 0.26 | 50.77 | |
| P 18. | Hepatic cirrhosis caused by hepatitis C | 1220 2009 0h | A | 9.56 | 0.14 | 68.29 | 40 |
| P 18. | | 1220 2009 3h | A | 16.08 | 0.26 | 81.85 | |
| P 18. | | 1220 2009 6h | A | 19.44 | 0.17 | 114.35 | |
| P 18. | | 1221 2009 24h | A | 17.35 | 0.20 | 86.75 | |
| P 43. | Burn | 0502 2010 0h | A | 2.59 | 0.48 | 5.40 | |
| P 43. | | 0502 2010 3h | A | 3.16 | 0.48 | 6.58 | |
| P 43. | | 0502 2010 6h | A | 4.05 | 0.51 | 7.94 | |
| P 43. | | 0503 2010 24h | | 1.28 | 0.42 | 3.05 | |
| P 43. | | 0504 2010 2d | | 4.29 | 0.29 | 14.79 | |
| P 43. | | 0505 2010 3d | A | 7.47 | 0.46 | 16.24 | |
| P 43. | | 0505 2010 3d | A | 8.64 | 0.32 | 27.00 | |
| P 49. | Sepsis | 0523 2010 0h | A | | 0.25 | | 17 |
| P 49. | | 0523 2010 6h | A | 2.07 | 0.48 | 4.31 | 17 |
| P 49. | | 0523 2010 12h | A | 2.36 | 0.43 | 5.49 | 17 |
| P 49. | | 0524 2010 | A | 3.76 | 0.40 | 9.40 | 20 |
| P 49. | | 0525 2010 3d | A | 1.49 | 0.30 | 4.97 | 17 |
| P 49. | | 0526 2010 4d | A | 3.16 | 0.30 | 10.50 | 19 |
| P 49. | | 0527 2010 5d | A | 3.60 | 0.22 | 16.35 | |
| P 50. | Hemobilia/Hepatic coma | 0607 2010 | A | 16.75 | 0.47 | 35.64 | 46 |
| P 50. | | 0607 2010 | A | 13.16 | 0.43 | 30. 60 | 36 |
| P 50. | | 0608 2010 | A | 10.60 | 0.31 | 35.33 | 38 |
| P 50. | | 0609 2010 | A | 12.33 | 0.20 | 61. 65 | 38 |
| P 54. | | 0717 2010 | A | 1.47 | 0.31 | 4.74 | |
| P 54. | | 0719 2010 | A | 1.55 | 0.30 | 5.17 | |
| P 54. | | 0720 2010 | A | 1.50 | 0.33 | 4.55 | |
| P 54. | | 0721 2010 | A | 1.46 | 0.38 | 3.84 | |
| P 54. | | 0722 2010 | A | 1.38 | 0.19 | 7.26 | |
| P 60. | Hepatic cirrhosis | 1006 2010 | A | 2.32 | 0.25 | 9.28 | 32 |
| P 60. | | 1007 2010 | A | 2.39 | 0.24 | 9.96 | 26 |
| P 60. | | 1009 2010 | A | 5.26 | 0.23 | 22.87 | 25 |

(continued)

| Group C (Severe case → Death) | | | | | | | |
|---|---|---|---|---|---|---|---|
| Patient ID | Disease name | Blood collection date(elapsed time) | Blood collection site | Lactic acid level (mM) | ATP level (mM) | A-LES | APACHE II score |
| P 63. | Sepsis | | A | 29.61 | 0.33 | 89.73 | 44 |

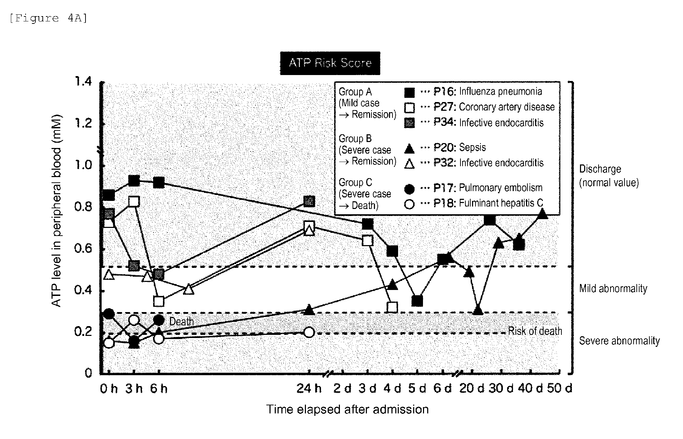

Figure 4A shows time-dependent changes in ATP levels in 3 patients of Nos. P16 (influenza pneumonia), P27 (coronary artery disease), and P34 (infective endocarditis) in Group A (taking a course from mild case to remission), 2 patients of Nos. P20 (sepsis) and P32 (infective endocarditis) in Group B (taking a course from severe case to remission), and 2 patients of Nos. P17 (pulmonary embolism) and P18 (fulminant hepatitis C) in Group C (taking a course from severe case to death) from among 43 patients. When monitored with ATP levels as an index, the patients taking a course to remission were discharged from the emergency intensive care unit because their ATP levels gradually increased and finally recovered to the normal value (0.52 mM or higher).

As shown in Figure 4A, the severity was assessed as severe abnormality when the ATP level was lower than 0.3 mM. Improvement in general status was observed, leading to remission, when the ATP level increased along with treatment. By contrast, a high mortality risk was confirmed when the ATP level remained lower than 0.3 mM for 6 to 24 hours or longer. The patient P32 had mild abnormality based on the ATP level at the time of admission, but was classified into severe patients from the A-LES value shown below and the general status.

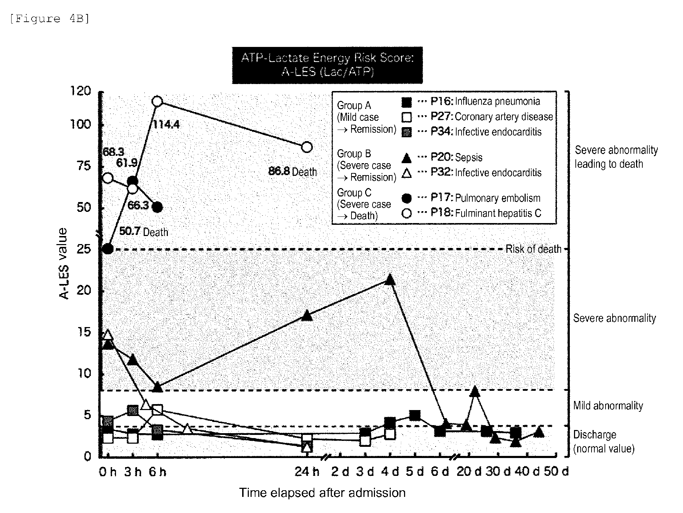

Results of monitoring with A-LES (Lac/ATP) values as an index instead of ATP levels are shown in Figure 4B. The severity of an illness was scored more extensively and reflected more accurately with the A-LES values as an index, which involved the level of the intermediate metabolite lactic acid of energy metabolism as a factor, than the ATP levels alone as an index.

The patients of Group A (taking a course from mild case to remission) mostly had A-LES values that fell within the normal range or did not exceed 8.0, and all exhibited A-LES values in the normal range (3.7 or lower) at the time of discharge. The patients of Group B (taking a course from severe case to remission) exhibited A-LES values exceeding 8.0 in the range corresponding to severe case equal to or lower than 25.0, when initially admitted. All the cases taking a course to remission exhibited A-LES values in the normal range (3.7 or lower) at the time of discharge, though various courses were observed depending on their illnesses. The patient P20 with sepsis will be shown as an example of the A-LES value that helps evaluate a treatment method. The A-LES value of this patient temporarily decreased in response to an antibiotic prescribed at the time of admission, but then rose continuously, leading to exacerbation. On the 4th day of admission, the antibiotic was changed to a new one selected from a bacterial sensitivity test. As a result, the condition was improved as the A-LES value rapidly decreased. Finally, the patient was discharged. This case shows that the A-LES value is useful in monitoring the effects of a therapeutic drug and a treatment method. On the other hand, the A-LES values in most of the severely ill patients in Group C who finally died as a result of exacerbation were already as high as higher than 25.0 at the time of admission and did not exhibit improvement to equal to or lower than 25.0 within 6 to 24 hours in spite of treatment. This demonstrated that only patients whose A-LES values fell within the normal range were discharged from the intensive care unit.

(Example 2) Comparison of movements in APACHE II scores and A-LES between at the time of admission into an emergency intensive care unit and at the time of discharge (or at the time of death)

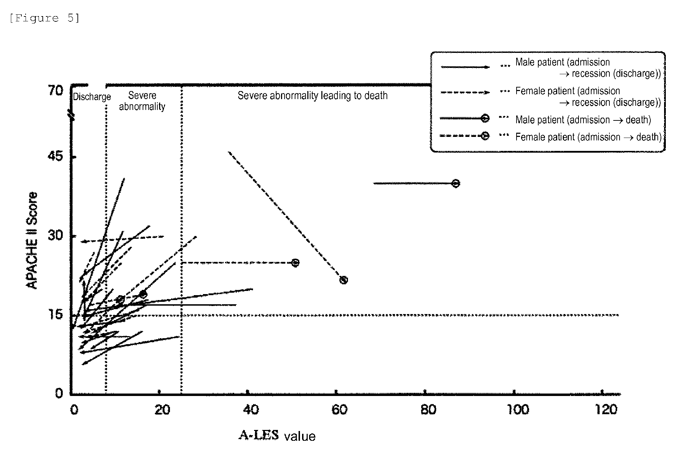

In this Example targeting 29 patients who were admitted into an emergency intensive care unit because of various diseases, movements in APACHE II scores and A-LES values in the patients between at the time of admission and at the time of discharge (or at the time of death) were compared after approval of the ethics committee of the University of Tokushima. The results are shown in Figure 5. The ATP levels and the lactic acid levels were measured by the same approaches as in Reference Examples 1 and 3, and A-LES values were calculated from the results (Figure 5).

The APACHE II scores decreased with decrease in the A-LES values in most of patients (16 males and 8 females) in a group taking a course from severe or mild case to remission. By contrast, the APACHE II scores of severely ill patients who finally died as a result of exacerbation remained high or decreased in some cases, whereas their A-LES values exhibited the tendency to rise along with the exacerbation of the disease conditions and well reflected the degree of increased severity. As is evident from these results, the A-LES value can not only serve as a real-time marker to reflect disease conditions but evaluate the degree of increased severity in more detail even in patients having high APACHE II scores.

(Example 3) ATP levels, lactic acid levels, A-LES values, ketone body levels (β-hydroxybutyric acid levels), and A-KES values (β-hydroxybutyric acid level /ATP level) in samples derived from healthy mice and severely ill mice infected with influenza virus

In this Example, ATP levels, lactic acid levels, A-LES values, ketone body levels (3-hydroxybutyric acid levels), and A-KES values (3-hydroxybutyric acid level/ATP level) in an experimental system using severely ill mice infected with influenza virus was shown as an example in which ATP levels and A-LES values can be used preferably in severity evaluation even using non-human vertebrate (test animal)-derived blood. Only 3-hydroxybutyric acid levels, not acetoacetic acid levels, were measured as the ketone body levels due to limitations in the amount of mouse blood necessary for the measurement. For this reason, the A-KES values were determined and evaluated from the 3-hydroxybutyric acid level/ATP level. In the viral infection test, each 4-week-old wild-type mouse (C57BL/6) was transnasally infected with 120 PFU of influenza virus (Influenza A/PR/8/34: H1N1). On the other hand, saline was transnasally administered instead of the virus to healthy mice as controls. On the 7th day (immediately before some of these mice died), blood was collected, and ATP levels, lactic acid levels, A-LES values, ketone body levels (3-hydroxybutyric acid levels), and A-KES values (β-hydroxybutyric acid level/ATP level) were determined (Figure 6).

The severely ill mice infected with influenza virus were confirmed to have decrease in ATP level and increase in lactic acid level, compared with the healthy mice. This showed a remarkable rise in A-LES value. These results demonstrated that the A-LES value was able to evaluate severity or therapeutic effects or the like even using samples from vertebrates (test animals) other than humans (test subjects). Since reference A-LES values for severity differ depending on animal species, the criteria in humans can be referred to but are not directly applied to the non-human animals. Also, the severely ill mice infected with influenza virus were confirmed to have decrease in ATP level and increase in ketone body level (3-hydroxybutyric acid level), compared with the healthy mice. This showed a remarkable rise in A-KES value (3-hydroxybutyric acid level/ATP level). This result demonstrated that the A-KES value also achieved evaluation of severity or therapeutic effects or the like. Since reference A-KES values for severity differ depending on the type of the ketone body level (e.g., the 3-hydroxybutyric acid level alone or the total of acetoacetic acid and 3-hydroxybutyric acid levels) or animal species.

As shown above, the blood ATP level was shown to reflect the "state of energy required for living" and also shown to serve as a novel index to represent the severity of an illness in real time. Furthermore, the value of lactic acid in blood that usually increases, during muscle fatigue or impaired oxygen utilization of tissues, as an intermediate metabolite of energy metabolism can be reevaluated as an A-LES value with blood ATP as a denominator and thereby indicated as a sensitive "energy risk score", showing the severity of an illness. Particularly, the A-LES value sensitively represents the severity of illnesses such as energy metabolism-related diseases, for example, infectious diseases, diabetes mellitus, metabolic diseases (e.g., mitochondrial encephalomyopathy), peripheral circulation insufficiency, CO poisoning, deficiency of energy metabolic enzymes, but can serve as a real-time risk marker for the severity of illnesses other than these diseases. It was also shown that the A-KES value, which is the ratio of the ketone body level in blood to the ATP level in blood, can also be reevaluated, thereby determining the severity of an illness.

**Industrial Applicability**

As described above in detail, the testing method of the present disclosure has enabled the severity of an illness to be assessed in real time by a more objective and convenient approach than the conventional APACHE II or SOFA score, on the basis of ATP levels, lactic acid levels, ketone body levels, the ratios thereof to the ATP levels (A-LES (Lac/ATP) or A-KES (Ketone/ATP) values) in samples. This testing method has been clearly demonstrated to be an unprecedented "method for assaying the severity of an illness". The testing method of the present invention brings such immeasurable benefits that it can be applied to clinical examinations, thereby finding an early sign of the increased severity of the illness in a patient and examining or evaluating the effects of a therapeutic strategy. Furthermore, "patients at a high risk" of developing a certain illness, for example, diabetic patients, obese persons, pregnant women, dialyzed patients, patients with chronic diseases who are reportedly patients at a high risk of having influenza infection, can be classified in detail on the basis of the A-LES or A-KES values, thereby elucidating the mechanism underlying increased severity and developing a treatment method.

The present invention can also be applied preferably to the diagnosis of the severity of an illness in humans and animals such as livestock.

**Claims**

1.    A method for testing the severity of an illness, that can determine the severity of an illness in real time, the method comprising the following steps A) to-D):

A) a step of measuring adenosine triphosphate level in a sample;
B) a step of measuring lactic acid level in the sample;
C) a step of calculating lactic acid level / adenosine triphosphate level which is a ratio of the lactic acid level measured in step B to the adenosine triphosphate level measured in step A; and
D) a step of using the ratio calculated in step C as an index for the severity of the illness.

2. The testing method according to claim 1, wherein measuring the level of adenosine triphosphate in a sample comprises:

1) a step of treating the sample with a solution comprising a phenol compound and extracting adenosine triphosphate in the sample to measure the level of adenosine triphosphate contained in the sample; and
2) a step of measuring the level of the extracted adenosine triphosphate using a reagent for adenosine triphosphate assay.

3. The testing method according to claim 2, wherein the solution comprising a phenol compound has a pH of 4 to 10.

4. The testing method according to claim 2 or 3, wherein the solution comprising a phenol compound further comprises a protein denaturant.

5. The testing method according to any one of claims 2 to 4, wherein the phenol compound is phenol.

6. The testing method according to any one of claims 1 to 5, wherein the sample is blood obtained from a test subject, and is assessed as abnormal when the ratio of the lactic acid level to the adenosine triphosphate level in the blood is higher than the upper limit 3.7 of its normal value.

7. The testing method according to claim 6, wherein the severity of an illness is assessed as mildly abnormal when the ratio of the lactic acid level to the adenosine triphosphate level in the blood is higher than the upper limit 3.7 of its normal value and equal to or lower than 8.0, and as severely abnormal when the ratio is higher than 8.0 and equal to or lower than 25.0, and as severely abnormal leading to death when a high value exceeding 25.0 continues for 6 hours or longer.

8. The testing method according to any one of claims 1 to 7, further comprising measuring a ketone body level in the sample.

9. The testing method according to claim 8, wherein ketone body level / adenosine triphosphate level which is a ratio of the ketone body level to the adenosine triphosphate level in the sample is used as an index for the severity of an illness.

10. The testing method according to claim 8 or 9, wherein the sample is blood obtained from a test subject, and is assessed as abnormal when the ratio of the ketone body level to the adenosine triphosphate level in the blood is higher than the upper limit 0.25 of its normal value.

**Patentansprüche**

1. Ein Verfahren zum Prüfen der Schwere einer Krankheit, das die Schwere einer Krankheit in Echtzeit messen kann, wobei das Verfahren die folgenden Schritte A) bis D) umfasst:

A) Messung des Adenosintriphosphat-Spiegels in einer Probe,
B) Messung des Milchsäure-Spiegels in einer Probe,
C) Berechnung des Milchsäure-Spiegels / Adenosintriphosphat-Spiegels, der ein Verhältnis des in Schritt B) gemessenen Milchsäure-Spiegels zu dem in Schritt A) gemessenen Adenosintriphosphat-Spiegel ist, und
D) Verwendung des berechneten Verhältnisses aus Schritt C als Kennzahl für die Schwere der Krankheit.

2. Das Prüfverfahren nach Anspruch 1, worin die Messung des Adenosintriphosphat-Spiegels in einer Probe

1) eine Behandlung der Probe mit einer Lösung, umfassend eine Phenolverbindung, und Extrahieren von Adenosintriphosphat aus der Probe, um den Spiegel von Adenosintriphosphat, das in der Probe enthalten ist, zu messen; und

2) eine Messung des Spiegels des extrahierten Adenosintriphosphats unter Verwendung eines Reagenzes für einen Adenosintriphosphat Assay,

umfasst.

3. Das Prüfverfahren nach Anspruch 2, worin die Lösung, die eine Phenolverbindung enthält, einen pH-Wert von 4 bis 10 aufweist.

4. Das Prüfverfahren nach Anspruch 2 oder 3, worin die Lösung, die eine Phenolverbindung enthält, ferner ein Proteindenaturierungsmittel enthält.

5. Das Prüfverfahren nach einem der Ansprüche 2 bis 4, worin die Phenolverbindung Phenol ist.

6. Das Prüfverfahren nach einem der Ansprüche 1 bis 5, worin die Probe Blut ist, das von einem Testsubjekt erhalten wurde und als abnormal bewertet wird, wenn das Verhältnis des Milchsäure-Spiegels zu dem Adenosintriphosphat-Spiegel im Blut höher ist als die Obergrenze 3,7 von seinem Normalwert.

7. Das Prüfverfahren nach Anspruch 6, worin die Schwere der Krankheit als milde abnormal beurteilt wird, wenn das Verhältnis des Milchsäure-Spiegels zu dem Adenosintriphosphat-Spiegel im Blut höher ist als die Obergrenze 3,7 von seinem Normalwert und gleich oder geringer ist als 8,0, und als schwerwiegend abnormal, wenn das Verhältnis höher als 8,0 und gleich oder niedriger als 25,0 ist, und als schwerwiegend abnormal mit Todesfolge, wenn ein hoher Wert, der 25,0 übersteigt, über 6 Stunden oder länger andauert.

8. Das Prüfverfahren nach einem der Ansprüche 1 bis 7, ferner umfassend die Messung eines Ketonkörper-Spiegels in der Probe.

9. Das Prüfverfahren nach Anspruch 8, worin der Ketonkörper-Spiegel / Adenosintriphosphat-Spiegel, der das Verhältnis des Ketonkörper-Spiegels zu dem Adenosintriphosphat-Spiegel ist, in einer Probe als eine Kennzahl für die Schwere einer Krankheit verwendet wird.

10. Prüfverfahren nach Anspruch 8 oder 9, worin die Probe Blut ist, das von einem Testsubjekt erhalten wurde, und als abnormal bewertet wird, wenn das Verhältnis des Ketonkörper-Spiegels zu dem Adenosintriphosphat-Spiegel im Blut höher ist als die Obergrenze 0,25 von seinem Normalwert.

**Revendications**

1. Procédé pour tester la gravité d'une maladie, qui peut déterminer la gravité d'une maladie en temps réel, le procédé comprenant les étapes suivantes A) à D) :

A) une étape qui consiste à mesurer le niveau d'adénosine triphosphate dans un échantillon ;
B) une étape qui consiste à mesurer le niveau d'acide lactique dans l'échantillon ;
C) une étape qui consiste à calculer le niveau d'acide lactique / le niveau d'adénosine triphosphate qui est un rapport du niveau d'acide lactique mesuré dans l'étape B sur le niveau d'adénosine triphosphate mesuré dans l'étape A ; et
D) une étape qui consiste à utiliser le rapport calculé dans l'étape C en tant qu'indice pour la gravité de la maladie.

2. Procédé de test selon la revendication 1, dans lequel la mesure du niveau d'adénosine triphosphate dans un échantillon comprend :

1) une étape qui consiste à traiter l'échantillon avec une solution comprenant un composé phénol et à extraire de l'adénosine triphosphate dans l'échantillon pour mesurer le niveau d'adénosine triphosphate contenue dans l'échantillon ; et
2) une étape qui consiste à mesurer le niveau de l'adénosine triphosphate extraite en utilisant un réactif pour un dosage d'adénosine triphosphate.

3. Procédé de test selon la revendication 2, dans lequel la solution comprenant un composé phénol a un pH allant de 4 à 10.

4. Procédé de test selon la revendication 2 ou 3, dans lequel la solution comprenant un composé phénol comprend en outre un dénaturant de protéines.

5. Procédé de test selon l'une quelconque des revendications 2 à 4, dans lequel le composé phénol est du phénol.

6. Procédé de test selon l'une quelconque des revendications 1 à 5, dans lequel l'échantillon est du sang obtenu à partir d'un sujet test, et est évalué comme étant anormal lorsque le rapport du niveau d'acide lactique sur le niveau d'adénosine triphosphate dans le sang est supérieur à la limite supérieure de 3,7 de sa valeur normale.

7. Procédé de test selon la revendication 6,
dans lequel la gravité d'une maladie est évaluée comme étant légèrement anormale lorsque le rapport du niveau d'acide lactique sur le niveau d'adénosine triphosphate dans le sang est supérieur à la limite supérieure de 3,7 de sa valeur normale et inférieur ou égal à 8,0, et comme étant sévèrement anormale lorsque le rapport est supérieur à 8,0 et inférieur ou égal à 25,0, et comme étant sévèrement anormale conduisant à la mort lorsqu'une valeur élevée dépassant 25,0 continue pendant 6 heures ou plus.

8. Procédé de test selon l'une quelconque des revendications 1 à 7, comprenant en outre la mesure d'un niveau de corps cétonique dans l'échantillon.

9. Procédé de test selon la revendication 8, dans lequel le niveau de corps cétonique / le niveau d'adénosine triphosphate qui est un rapport du niveau de corps cétonique sur le niveau d'adénosine triphosphate dans l'échantillon est utilisé en tant qu'indice pour la gravité d'une maladie.

10. Procédé de test selon la revendication 8 ou 9, dans lequel l'échantillon est du sang obtenu à partir d'un sujet test, et est évalué comme étant anormal lorsque le rapport du niveau de corps cétonique sur le niveau d'adénosine triphosphate dans le sang est supérieur à la limite supérieure de 0,25 de sa valeur normale.

[Figure 1]

[Figure 2]

[Figure 3]

[Figure 4A]

[Figure 4B]

Time elapsed after admission

[Figure 5]

[Figure 6]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009096429 A

### Non-patent literature cited in the description

- **KNAUS WA ; DRAPER EA ; WAGNER DP ; ZIMMERMAN JE.** A severity of disease classification system. *Critical Care Medicine,* 1985, vol. 13, 818-829
- **VINCENT JL ; MORENO R ; TAKALA J ; WILLATTS S ; DE MENDONCA A ; BRUINING H ; REINHART CK ; SUTER PM ; THIJS LG.** The SOFA (Sepsis-related Organ Failure Assessment) score to describe organ dysfunction/failure. On behalf of the Working Group on Sepsis-Related Problems of the European Society of Intensive Care Medicine. *Intensive Care Medicine,* 1996, vol. 7, 707-710
- *Critical Care Medicine,* 1985, vol. 13, 818-829
- *Intensive Care Medicine,* 1996, vol. 7, 707-710
- **HARUMI NISHIGAYA et al.** *Japanese Journal of Medical Technology,* 1996, vol. 45 (3), 353
- **YUTAKA HARANOU et al.** *Japanese Journal of Clinical Medicine,* 1990, vol. 48, 323-333